# EUROPÄISCHE PATENTSCHRIFT

(11) **EP 2 393 795 B1**
(45) Veröffentlichungstag und Bekanntmachung des Hinweises auf die Patenterteilung: **26.12.2012**
(21) Anmeldenummer: 10703446.4
(22) Anmeldetag: 04.02.2010
(51) Int. Cl.: C07D 303/08, C07D 303/14, C07D 405/06

(54) **VERFAHREN ZUR HERSTELLUNG VON HYDROXYMETHYLDIPHENYLOXIRANEN UND ENTSPRECHENDEN 1-AZOLYLMETHYL-1,2-DIPHENYLOXIRANEN**
METHOD FOR PRODUCING HYDROXYMETHYL DIPHENYLOXIRANES AND CORRESPONDING 1-AZOLYLMETHYL-1,2-DIPHENYLOXIRANES
PROCÉDÉ DE PRODUCTION D'HYDROXYMÉTHYLDIPHÉNYLOXIRANES ET DES 1-AZOLYLMÉTHYL-1,2-DIPHÉNYLOXIRANES CORRESPONDANTS

(30) Priorität: 05.02.2009 EP 09152176
(43) Veröffentlichungstag der Anmeldung: 14.12.2011
(73) Patentinhaber: BASF SE, 67056 Ludwigshafen (DE)
(72) Erfinder: NOACK, Rainer, 04932 Großthiemig (DE); PALM, Clemens, 01109 Dresden (DE); GRÖNING, Carsten, 68259 Mannheim (DE); LIPOWSKY, Gunter, 68526 Ladenburg (DE)
(86) Internationale Anmeldenummer: PCT/EP2010/051380
(87) Internationale Veröffentlichungsnummer: WO 2010/089353

(56) Entgegenhaltungen:
- EP-A- 0 334 035
- EP-A- 0 352 675
- H. E. ZIMMERMAN ET AL.: "Overlap Control of Carbanionoid Reactions. I. Stereoselectivity in Alkaline Epoxidation" JOURNAL OF THE AMERICAN CHEMICAL SOCIETY, Bd. 81, 1959, Seiten 108-116, XP002525171
- T. BURGER ET AL.: "Synthesis of Four (14)C-Isotopomers of Epoxiconazole, a New Triazole Fungicide" JOURNAL OF LABELLED COMPOUNDS AND RADIOPHARMACEUTICALS, Bd. 38, Nr. 2, 1996, Seiten 174-178, XP002525172

## Beschreibung

Die vorliegende Erfindung betrifft ein Verfahren zur Herstellung von 1-Hydroxymethyl-1,2-diphenyloxiranen aus 2,3-Diphenylpropenalen durch Epoxidierung und Reduktion. Die Hydroxymethyldiphenyloxirane stellen wertvolle Zwischenprodukte für die Herstellung von 1-Azolylmethyl-1,2-diphenyloxiranen dar, aus denen letztere durch Einführung der Azolylgruppe leicht hergestellt werden können. Dementsprechend betrifft die Erfindung auch ein Verfahren zur Herstellung von 1-Azolylmethyl-1,2-diphenyloxiranen.

Die industrielle Bedeutung von Azolylmethyldiphenyloxiranen ist enorm. Insbesondere in den Bereichen Pharmazie und Pflanzenschutz finden sich zahlreiche Wirkstoffe aus dieser Strukturklasse. So seien an dieser Stelle beispielsweise aus dem Bereich des Pflanzenschutzes 1-(1,2,4-Triazol-1-ylmethyl)-1,2-diphenyloxirane wie Epoxiconazole mit fungiziden und teilweise auch wachstumsregulierenden Eigenschaften genannt.

Verfahren zur Herstellung von Hydroxymethyldiphenyloxiranen sind allgemein bekannt, z.B. durch Epoxidierung von Diphenylpropenolen mit Peroxiden und speziellen Katalysatoren (Übersicht: A. Pfenninger, "Asymmetric Epoxidation of Allylic Alcohols: The Sharpless Epoxidation", Synthesis 1986, 89). In der Regel sind aber die bevorzugten in trans-Stellung zu den Phenylgruppen substituierten Diphenylpropenole nicht verfügbar oder nur schwer zugänglich, oder die Synthesebedingungen sind technisch nicht mit vertretbarem Aufwand realisierbar.

In guten Ausbeuten gelingt die Herstellung der Hydroxymethyldiphenyloxirane bekanntermaßen, indem man ein entsprechend substituiertes 2,3-Diphenylpropenal durch Epoxidierung zum 1-Formyl-1,2-diphenyloxiran umsetzt und dieses dann zum 1-Hydroxymethyl-1,2-diphenyloxiran reduziert. So finden sich in EP 330 132, EP 332 073, EP 334 035, EP 352 673, EP 352 675 und EP 421 125 Beispiele für die Epoxidierung von substituierten 2,3-Diphenylpropenalen, z.B. 2-(4-Fluorphenyl)-3-(2-chlorphenyl)-propenal, zu den entsprechenden Formyloxiranen und ihre anschließende Reduktion zu den 1-Hydroxymethyl-1,2-diphenyloxiranen.

Im Zuge der Epoxidierung und Reduktion entstehen allerdings Nebenprodukte. Insbesondere beim Einsatz substituierter 2,3-Diphenylpropenale, wie sie beispielsweise für die Herstellung von Epoxiconazolen gebraucht werden, fällt ein bestimmter Typus eines - im Falle der vorstehend erwähnten Umsetzung von 2-(4-Fluorphenyl)-3-(2-chlorphenyl)-propenal die Struktur A 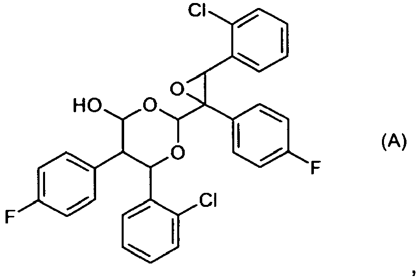 aufweisenden - lipophilen Nebenproduktes in erheblicher Menge an. Besonders störend ist, dass das Nebenprodukt auch im eigentlichen Wertprodukt, dem Azolylmethyldiphenyloxiran, wiederzufinden ist, wenn man das verunreinigte Hydroxymethyldiphenyloxiran in gewohnter Weise zum gewünschten Azolylmethyldiphenyloxiran umsetzt. Da das Nebenprodukt die Eigenschaften des Azolylmethyldiphenyloxirans beeinträchtigt, ist eine aufwendige Reinigung des Wertproduktes erforderlich.

Eine Aufgabe der vorliegenden Erfindung ist es daher, ein Verfahren zur Herstellung von Hydroxymethyldiphenyloxiranen zur Verfügung zu stellen, die weniger des eingangs bezeichneten lipophilen Nebenproduktes enthalten. Ein Gehalt von weniger als 1 Gew.-%, vorzugsweise von weniger als 0,6 Gew.-%, bezogen auf das Wertprodukt ist erstrebenswert.

Die Aufgabe wird gelöst durch ein Verfahren nach Anspruch 1. Besondere Ausgestaltungen des Verfahrens sind Gegenstand der Patentansprüche 2 bis 5, 10 und 13 bis 15.

Dabei epoxidiert man ein 2,3-Propenal der Formel (V): 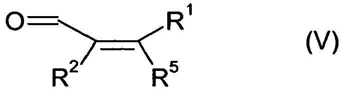 worin
R¹, R² unabhängig voneinander für Phenyl stehen, wobei jeder Phenylrest unabhängig voneinander 1 bis 3 Substituenten aufweisen kann, die ausgewählt sind unter Halogen, Cyano, Nitro, C₁-C₄-Alkyl, Halogen-C₁-C₄-alkyl, Hydroxy, C₁-C₄-Alkoxy, Halogen-C₁-C₄-alkoxy, Mercapto, C₁-C₄-Alkylthio, Halogen-C₁-C₄-alkylthio, Sulfinyl, Sulfonyl, C₁-C₄-Alkylsulfonyl, Phenylsulfonyl, Phenyl, Phenoxy, Amino, C₁-C₄-Alkylamino, Di-C₁-C₃-alkylamino, -NHCO-C₁-C₃-Alkyl, -NHCOO-C₁-C₄-Alkyl, - COO-C₁-C₄-Alkyl und -CONH-C₁-C₄-Alkyl, wobei jeder der Substituenten Phenyl, Phenoxy und Phenylsulfonyl unabhängig voneinander 1 bis 3 Substituenten aufweisen kann, die ausgewählt sind unter Halogen und C₁-C₄-Alkyl; und
R⁵ für Wasserstoff oder Methyl steht,
zu einem Formyloxiran der Formel (IV): 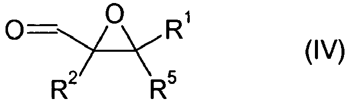 worin R¹, R² und R⁵ wie vorstehend definiert sind,
und reduziert das Formyloxiran der Formel (IV) zu einem Hydroxymethyloxiran der Formel (III): 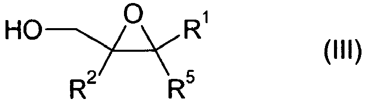 worin R¹, R² und R⁵ wie vorstehend definiert sind,
wobei das Verfahren dadurch gekennzeichnet ist, dass man mit der Reduktion beginnt, solange die Menge an eingesetzter Verbindung der Formel (V) in dem Reaktionsgemisch noch mindestens etwa 2 mol-% beträgt.

Demnach ist das erfindungsgemäße Verfahren dadurch gekennzeichnet, dass mit der Reduktion bereits begonnen wird, bevor das 2,3-Propenal der Formel (V) vollständig umgesetzt ist.

Das resultierende Hydroxymethyloxiran der Formel (III) enthält wesentlich weniger des eingangs erwähnten lipophilen Nebenprodukts. Vorzugsweise liegt der Gehalt des Nebenprodukts bei weniger als 1 Gew.-% und insbesondere bei weniger als 0,6 Gew.-% oder 0,5 Gew.-%. Ein solches Ergebnis war nicht zu erwarten.

Das erfindungsgemäße Verfahren findet, wie eingangs beschrieben, vielfältige Anwendung, da die resultierenden Hydroxymethyloxirane wertvolle Zwischenprodukte darstellen. So kann das erfindungsgemäße Verfahren auch Teil eines Verfahrens nach Anspruch 6, nämlich eines Verfahrens zur Herstellung von Azolylmethyloxiranen der Formel (I): 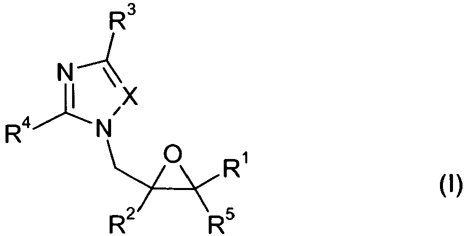 sein, worin
X für N oder CH steht;
R³, R⁴ unabhängig voneinander für Wasserstoff, Halogen, C₁-C₆-Alkyl, Mercapto, -S-CN, C₁-C₆-Alkylthio, C₂-C₆-Alkenylthio, C₆-C₁₂-Aryl-C₁-C₃-alkylthio oder C₆-C₁₂-Arylthio stehen, wobei C₂-C₆-Alkenylthio 1 bis 3 Substituenten aufweisen kann, die ausgewählt sind unter Halogen, C₁-C₄-Alkyl und Halogen-C₁-C₄-alkyl, und das Aryl in C₆-C₁₂-Aryl-C₁-C₃-alkylthio 1 bis 5 Substituenten aufweisen kann, die ausgewählt sind unter Halogen, C₁-C₄-Alkyl und Halogen-C₁-C₄-alkyl; und
R¹, R² und R⁵ eine der hier angegebenen Bedeutungen aufweisen.

Besondere Ausgestaltungen des Verfahrens sind Gegenstand der Patentansprüche 7 bis 15.

Ein solches Verfahren beinhaltet als zusätzlichen Schritt die Einführung der Azolylgruppe in die Verbindung der Formel (III), wozu man in der Regel die Hydroxygruppe durch eine geeignete nukleofuge Gruppe ersetzt und dann mit der gewünschten Azolylverbindung umsetzt.

Die Edukte für die Epoxidierungsreaktion, d.h. die 2,3-Diphenylpropenale der Formel (V), lassen sich in an sich bekannter Weise herstellen..Beispielsweise kann man passende Phenylglyoxal-O,O-acetale mit (a) Benzylphosphonsäuredialklylestern nach Horner, Wadsworth und Emmons (W.S. Wadsworth, Synthetic Applications of Phosphoryl-Stabilized Anions, Org. Reactions 25, 73 (1977)); (b) Benzyltriphenylphosphoniumhalogeniden nach Wittig (M. Ogata et al., Eur. J. Med. Chem. 24 (1989) 137); oder (c) Benzylmagnesiumhalogeniden nach Grignard analog W. Madlung u. M. E. Oberwegner, Chem. Ber. 65, 936 (1936) umsetzen. Weiterhin steht für die Herstellung der 2,3-Diphenylpropenale als Variante d) die Aldolkondensation von Arylalkylaldehyden mit Arylaldehyden nach WO 2005056498 A2 zur Verfügung, die leicht zu epoxidierende, in cis-Stellung zu den Phenylgruppen substituierte 2,3-Diphenylpropenale liefert.

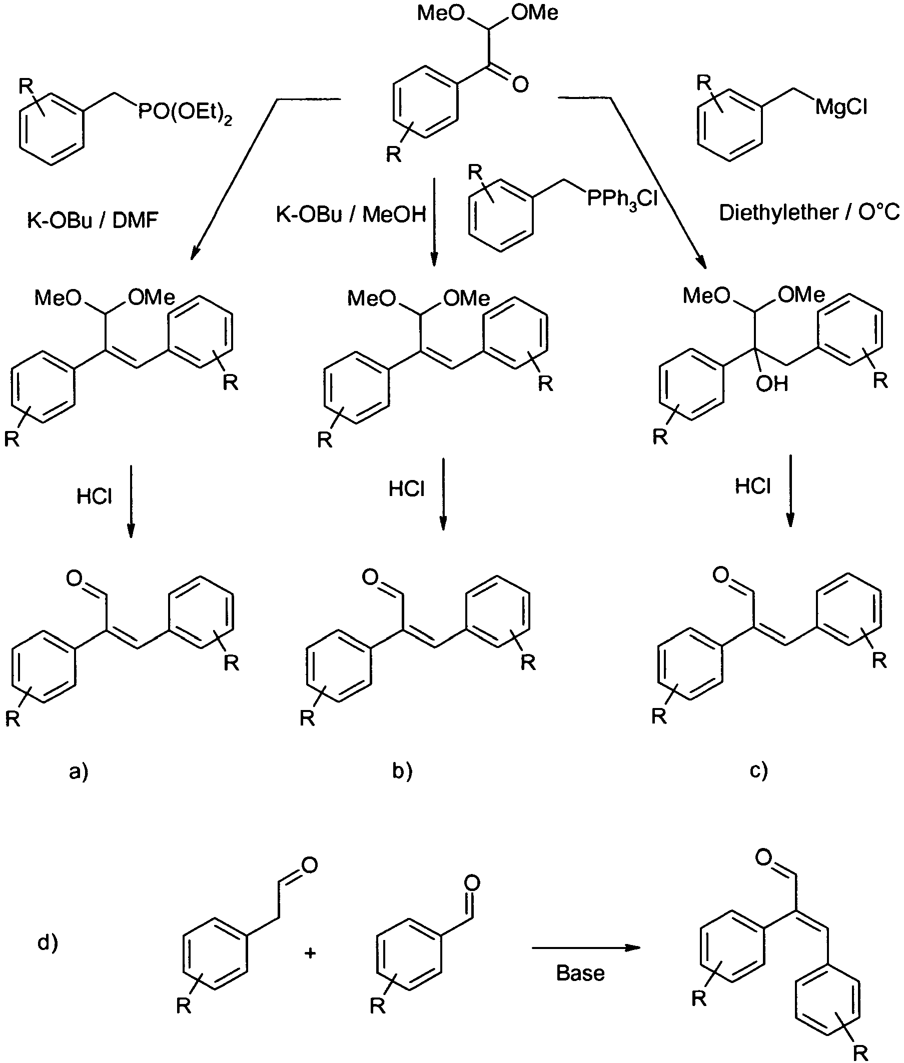

Die Epoxidierung kann in an sich bekannter Weise erfolgen.

Zu üblichen Oxidationsmitteln für die Epoxidation gehören Hydroperoxide, z.B. Wasserstoffperoxid, tert-Butylhydroperoxid, Cumylhydroperoxid, tert-Amylhydroperoxid und Tritylhydroperoxid, molekularer Sauerstoff, Percarbonate, Perborate und Peroxycarbonsäuren, wie Perbenzoesäure, meta-Chlorperbenzoesäure, 4-Nitroperbenzoesäure, Monoperphthalsäure, Peressigsäure, Perpropionsäure, Permaleinsäure, Monoperbernsteinsäure und Trifluorperessigsäure, und außerdem die Salze, insbesondere die Alkali- oder Erdalkalisalze, der Percarbonsäuren, Perborsäuren und Persäuren, wie Natriumpercarbonat, Natriumperborat oder Kaliumperoxomonosulfat. Bevorzugt sind die sterisch anspruchsvollen Hydroperoxide, wie tert-Butylhydroperoxid, die eine stereoselektive Bildung der bevorzugten trans-Oxirane bewirken.

Das Oxidationsmittel wird in der Regel wenigstens äquimolar und bevorzugt im Überschuss zum 2,3-Propenal der Formel (V) eingesetzt. Vorzugsweise beträgt das Molverhältnis von Oxidationsmittel zum 2,3-Propenal der Formel (V) 3:1 bis 1:1, besonders bevorzugt 2:1 bis 1:1 und insbesondere 1,5:1 bis 1:1. Sterisch anspruchsvolle Hydroperoxide, wie tert-Butylhydroperoxid, verwendet man bevorzugt in einem Molverhältnis von 1,2:1 bis 1:1.

Vorzugsweise erfolgt die Epoxidierung bei einem pH-Wert im basischen Bereich, z.B. bei einem pH von 7,1 bis 14, bevorzugt von 10 bis 13. Zur Einstellung des gewünschten pH-Werts setzt man dem Reaktionsmedium allgemein eine geeignete Base zu. Geeignete Basen sind beispielsweise Alkali- und Erdalkalihydroxide, wie Natrium-, Kalium-, Magnesium- oder Calciumhydroxid, Alkali- und Erdalkalicarbonate, wie Natrium-, Kalium-, Magnesium- oder Calciumcarbonat, und insbesondere Alkali- und Erdalkalihydrogencarbonate, wie Natrium-, Kalium-, Magnesium- oder Calciumhydrogencarbonat. Das Einstellen des basischen pH-Wertes hat Katalysatorfunktion, weswegen die zuzusetzende Base hier auch als Katalysator bezeichnet wird.

Die Base wird in einer solchen Menge eingesetzt, dass das Reaktionsmedium einen pH von wenigstens 7,1, z.B. 7,1 bis 14, bevorzugt wenigstens 10, z.B. 10 bis 13, aufweist. Bei der Verwendung von Wasserstoffperoxid wird die Base in einer solchen Menge eingesetzt, dass das Wasserstoffperoxid vorzugsweise vollständig deprotoniert wird (zu HOO⁻).

Die Epoxidierung kann sowohl in einem wasserhaltigen als auch in einem nicht wässrigen Medium erfolgen. Wasserhaltige Systeme können insbesondere dann von Vorteil sein, wenn anorganische Basen eingesetzt werden. Bei der Verwendung organischer Basen, die in organischen Lösungsmitteln löslich sind, ist es bevorzugt, die Epoxidierung in einem nicht wässrigen Medium durchzuführen. Geeignete organische Lösungsmittel in diesem Fall sind Lösungsmittel, die sich in der Epoxidierungsreaktion inert verhalten. Beispiele für geeignete organische Lösungsmittel sind C₁-C₄-Alkanole, wie Methanol, Ethanol, Propanol, Isopropanol und die Butanole, zyklische und offenkettige Ether, wie Tetrahydrofuran, Dioxan, Diethylether, Dipropylether, Diisopropylether, Dibutylether, tert-Butylmethylether und dergleichen, halogenierte Kohlenwasserstoffe, wie Methylenchlorid, Chloroform oder Tetrachlorkohlenstoff, aromatische Kohlenwasserstoffe, wie Benzol, Toluol, Nitrobenzol, Chlorbenzol, Dichlorbenzol und die Xylole, Carbonsäurederivate, wie Dimethylformamid und Ethylacetat, Nitrile, wie Acetonitril und Propionitril, und Dimethylsulfoxid.

Die Durchführung der Epoxidierung erfolgt vorzugsweise so, dass das 2,3-Diphenylpropenal der Formel (V) vorgelegt wird und mit dem Oxidationsmittel und gegebenenfalls der Base versetzt wird. Die Zugabe des Oxidationsmittels und der Base kann dabei sowohl getrennt als auch gemeinsam, auf einmal oder vorzugsweise in Portionen erfolgen. Insbesondere kann man zunächst die Base vollständig und anschließend das Oxidationsmittel portionsweise zugeben.

Die Reaktionstemperatur während der Epoxidierung beträgt in der Regel -20 bis +80°C, bevorzugt -0 bis 60°C und insbesondere 20°C bis 35°C.

Das Reaktionsprodukt aus der Epoxidierung wird erfindungsgemäß nicht isoliert, sondern es wird das Reaktionsgemisch noch während der laufenden Epoxidierung der Reduktion unterworfen. Dazu kann man den Verlauf der Epoxidierungsreaktion verfolgen, beispielsweise anhand des Umsatzes an Edukt, d.h. des 2,3-Diphenylpropenals der Formel (V).

Um der Bildung des eingangs genannten lipophilen Nebenprodukts gegenzusteuern, beginnt man erfindungsgemäß mit der Reduktion, solange noch mindestens etwa 2, 3, 4 oder 5 mol-%, in besonderen Fällen noch mindestens etwa 7,5 mol-% und unter Umständen noch mindestens etwa 10 mol-% des anfänglich in die Epoxidierungsreaktion eingetragenen Eduktes, d.h. des 2,3-Diphenylpropenals der Formel (V), im Reaktionsgemisch vorhanden sind, sprich noch nicht abreagiert haben. Andererseits ist es insbesondere mit Blick auf die mögliche Bildung von Nebenprodukten aus der Reduktion der Diphenylpropenale und dem damit einhergehenden Verlust an Gesamtausbeute zweckmäßig, mit der Reduktion erst dann zu beginnen, wenn die Epoxidierungsreaktion bereits zu einem gewissen Grad fortgeschritten ist. So kann in der Regel mit der Reduktion begonnen werden, wenn mehr als etwa 80 mol-%, vorzugsweise mehr als etwa 85 mol-% und insbesondere mehr als etwa 90 mol-% des anfänglich in die Epoxidierungsreaktion eingetragenen 2,3-Diphenylpropenals der Formel (V) abreagiert haben, d.h. die Menge an 2,3-Diphenylpropenal im Reaktionsgemisch weniger als 20 mol-%, vorzugsweise weniger als etwa 15 mol-% und insbesondere weniger als etwa 10 mol-% des anfänglich in die Epoxidierungsreaktion eingetragenen 2,3-Diphenylpropenals der Formel (V) beträgt. Berücksichtigt man die beiden gegenläufigen Bestrebungen einer möglichst vollständigen Unterdrückung des Nebenproduktes A und einer möglichst hohen Gesamtausbeute ist eine Reaktionsführung von besonderem Vorteil, bei der mit der Reduktion dann begonnen wird, solange noch etwa 2,5 mol-% bis 15 mol-%, vorzugsweise noch etwa 3 mol-% bis 12 mol-%, und insbesondere noch etwa 5 mol-% bis 10 mol-% des anfänglich in die Epoxidierungsreaktion eingetragenen Eduktes, d.h. des 2,3-Diphenylpropenals der Formel (V), im Reaktionsgemisch vorhanden sind.

Die Menge an 2,3-Diphenylpropenal im Reaktionsgemisch lässt sich in an sich bekannter Weise bestimmen, zum Beispiel durch Hochdruckflüssigchromatographie (HPLC), mit der der Reaktionsablauf in allen Schritten verfolgt werden kann.

Die Reduktion gelingt beispielsweise mit komplexen Hydriden oder nichtkomplexen Metall- und Halbmetallhydriden. Unter komplexen Hydriden versteht man im Allgemeinen geladene Metallkomplexe, die wenigstens einen Hydrid-Liganden enthalten. Beispiele hierfür sind Lithiumaluminiumhydrid (LiAlH₄), LiAlH(O-*tert*-butyl)₃, LiAIH(O-methyl)₃, NaAlEt₂H₂, Natriumborhydrid (NaBH₄) und dergleichen. Beispiele für nichtkomplexe Metall- und Halbmetallhydride sind Borane, wie BH₃, 9-BBN (9-Borabicyclo[3.3.1]nonan) und Disiamylboran, AlH₃, DIBAL-H (AlH(isobutyl)₂) und dergleichen.

Bevorzugte Reduktionsmittel sind die oben genannten komplexen Hydride und nichtkomplexen Metall- und Halbmetallhydride, von denen die Alkalimetallborhydride, z.B. Natriumborhydrid, besonders bevorzugt sind.

Das Reduktionsmittel wird in der Regel wenigstens äquimolar und besonders bevorzugt im Überschuss zum 2,3-Propenal der Formel (V) eingesetzt. Vorzugsweise beträgt das Molverhältnis von Reduktionsmittel zum 2,3-Propenal der Formel (V) 3:1 bis 1:1, besonders bevorzugt 2:1 bis 1:1 und insbesondere 1,5:1 bis 1:1.

Vorzugsweise erfolgt die Reduktion bei einem pH-Wert im basischen Bereich, z.B. bei einem pH von 7,1 bis 14, bevorzugt von 10 bis 13. Zur Einstellung des gewünschten pH-Werts setzt man dem Reaktionsmedium allgemein eine geeignete Base zu. Geeignete Basen sind beispielsweise Alkali- und Erdalkalihydroxide, wie Natrium-, Kalium-, Magnesium- oder Calciumhydroxid, Alkali- und Erdalkalicarbonate, wie Natrium-, Kalium-, Magnesium- oder Calciumcarbonat, Alkalimetallsalze von schwachen Säuren, wie Borate, und organische Basen, wie quarternäre Ammoniumhydroxide und spezielle tert. Amine, wie Diazabicyclooktan (DABCO), Diazabicycloundecen (DBU), Pentamethylguanidin oder cyclische Phosphoranbasen, wie BEMP. Das Einstellen des basischen pH-Wertes hat Katalysatorfunktion, weswegen die zuzusetzende Base hier auch als Katalysator bezeichnet wird.

Die Base wird in einer solchen Menge eingesetzt, dass das Reaktionsmedium einen pH von wenigstens 7,1, z.B. 7,1 bis 14, bevorzugt wenigstens 10, z.B. 10 bis 13, aufweist. Dabei ist allerdings zu beachten, dass zu hohe Konzentrationen an starken Basen, wie NaOH, KOH oder quarternären Ammoniumhydroxiden, zur verstärkten Bildung des eingangs beschriebenen Nebenproduktes führen.

Da gebildetes Formyloxiran erfindungsgemäß nicht isoliert wird, findet die Reduktion in dem für die Epoxidierung verwendeten Reaktionsmedium statt. Es kann aber zweckmäßig sein, vor, mit oder nach der Zugabe des Reduktionsmittels weiteres Lösungsmittel zuzusetzen. Dies kann ein anderes Lösungsmittel sein, als das für die Epoxidierung verwendete, und dazu dienen, das Reaktionsgemisch zu verdünnen und/oder Bedingungen einzustellen, die für die Bildung und/oder Gewinnung des Hydroxymethyloxirans günstig sind. Geeignete Lösungsmittel sind beispielsweise aliphatische Kohlenwasserstoffe, vorzugsweise mit 5 bis 8 Kohlenstoffatomen, wie Pentan, Cyclopentan, Hexan, Cyclohexan, Heptan, Octan oder Cyclooctan, oder technische Alkan- oder Cycloalkangemische, aromatische Kohlenwasserstoffe, wie Benzol, Toluol und die Xylole, aliphatische acyclische und cyclische Ether, vorzugsweise mit 4 bis 8 Kohlenstoffatomen, wie Diethylether, Methyl-tert-butylether, Ethyl-tert-butylether, Dipropylether, Diisopropylether, Dibutylether, Tetrahydrofuran oder Dioxan, oder Gemische der vorgenannten Lösungsmittel. Besonders bevorzugt werden die vorgenannten Ether oder aromatischen Kohlenwasserstoffe eingesetzt.

Die Durchführung erfolgt vorzugsweise so, dass gegebenenfalls weiteres Lösungsmittel zugegeben und mit dem Reduktionsmittel und gegebenenfalls der Base versetzt wird. Die Zugabe des Reduktionsmittels und der Base kann dabei sowohl getrennt als auch gemeinsam, auf einmal oder vorzugsweise in Portionen erfolgen. Insbesondere kann man zunächst die Base vollständig und anschließend das Reduktionsmittel portionsweise zugeben.

Die Reaktionstemperatur während der Reduktion beträgt in der Regel -20 bis +80°C, bevorzugt -0 bis 60°C und insbesondere 20°C bis 35°C.

Die Aufarbeitung des Reaktionsgemischs aus der Reduktionsreaktion kann in üblicher Weise erfolgen, beispielsweise durch Desaktivieren von nicht umgesetztem Reduktionsmittel, z.B. durch Versetzen des Reaktionsgemisch mit einem protischen Lösungsmittel, wie Wasser, oder einem C₁-C₃-Alkohol, wie Methanol, Ethanol, Propanol oder Isopropanol, und anschließendes Aufreinigen, beispielsweise durch Extraktion, Chromatographie und dergleichen.

Zur weiteren Umsetzung des Hydroxymethyloxirans kann auch wegen der hohen Reinheit des Produkts die nach Extraktion und Waschung erhaltene Lösung des Hydroxymethyloxirans direkt verwendet werden.

So kann die eingangs beschriebene Herstellung von Azolylmethyloxiranen der Formel (I) beispielsweise dadurch erfolgen, dass man eine Verbindung der Formel (II) 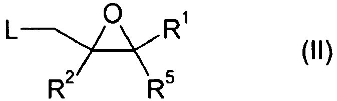 worin R¹, R² und R⁵ wie hier definiert sind und L für eine nukleophil substituierbare Abgangsgruppe steht,
mit einer Verbindung der Formel (VI) 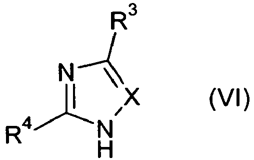 worin R³, R⁴ und X wie hier definiert sind,
oder mit einem Basenadditionssalz der Verbindung der Formel (VI) umsetzt.

Weitere Ausführungen hierzu finden sich beispielsweise in EP 0 352 675 A2, worauf hiermit in vollem Umfang Bezug genommen wird.

Demnach kann die Umsetzung in Gegenwart einer Base, eines Lösungs- oder Verdünnungsmittels und/oder unter Zusatz eines Reaktionsbeschleunigers bei Temperaturen zwischen 10 und 120°C erfolgen.

Zu den geeigneten Lösungs- und Verdünnungsmitteln gehören Ketone wie Aceton, Methylethylketon oder Cyclohexanon, Nitrile wie Acetonitril oder Propionitril, Alkohole wie Methanol, Ethanol, iso-Propanol, n-Butanol oder Glycol, Ester wie Essigsäureethylester, Essigsäuremethylester oder Essigsäurebutylester, Ether wie Tetrahydrofuran, Diethylether, Dimethoxyethan, Dioxan oder Diisopropylether, Amide wie Dimethylformamid, Dimethylacetamid oder N-Methylpyrrolidon ferner Dimethylsulfoxid, Sulfolan oder entsprechende Gemische.

Geeignete Basen, die gegebenenfalls auch als säurebindende Mittel bei der Reaktion verwendet werden können, sind beispielsweise Alkalimetallhydroxide wie Lithium-, Natrium- oder Kaliumhydroxid, Alkalimetallcarbonate wie Natrium-, Kalium- oder Cäsiumcarbonat oder Natrium-, Kalium- oder Cäsiumhydrogencarbonat, Pyridin oder 4-Dimethylaminopyridin. Es können aber auch andere übliche Basen verwendet werden. Als Reaktionsbebschleuniger kommen vorzugsweise Metallhalogenide wie Natriumjodid oder Kaliumjodid, quaternäre Ammoniumsalze wie Tetrabutylammoniumchlorid, - bromid, -jodid oder-hydrogensulfat, Benzyltriethylammoniumchlorid oder -bromid, oder Kronenether wie 12-Krone-4, 15-Krone-5, 18-Krone-6, Dibenzo-18-Krone-6 oder Dicyclohexano-18-Krone-6 in Frage.

Die Umsetzung wird z.B. bei Temperaturen zwischen 20 und 150°C, drucklos oder unter Druck, kontinuierlich oder diskontinuierlich durchgeführt.

Wird ein Basenadditionssalz der Verbindung der Formel (VI) mit einem Metallkation eingesetzt, ist es zweckmäßig, die Reaktion in Gegenwart eines Lösungs- oder Verdünnungsmittels und unter Zusatz einer starken anorganischen oder organischen Base bei Temperaturen zwischen -10 und 120°C durchzuführen. Zu den bevorzugten Lösungs- und Verdünnungsmitteln gehören in diesem Fall Amide wie Dimethylformamid, Diethylformamid, Dimethylacetamid, Diethylacetamid, N-Methylpyrrolidon, Hexamethylphosphortriamid, Sulfoxide wie Dimethylsulfoxid und schliesslich Sulfolan. Geeignete starke Basen, die gegebenenfalls auch als säurebindende Mittel bei der Reaktion verwendet werden können, sind beispielsweise Alkalihydride wie Lithium-, Natrium- und Kaliumhydrid, Alkaliamide wie Natrium- und Kaliumamid, ferner Natrium- oder Kalium-tert.-butoxid, Lithium-, Natrium- oder Kalium-triphenylmethyl und Naphthalinlithium, -natrium oder -kalium.

Verbindungen der Formel (II), worin L für eine nukleophil substituierbare Abgangsgruppe wie Halogen, C₁-C₆-Alkyl-SO₂-O- oder Aryl-SO₂-O- steht, sind beispielsweise dadurch erhältlich, dass man in Verbindungen der Formel (III) die CH₂OH-Gruppe in eine geeignete Abgangsgruppe überführt, beispielsweise in eine Gruppe CH₂L, worin L einer der angegebenen Bedeutungen hat. Die Überführung von Alkoholfunktionen in Abgangsgruppen ist allgemein bekannt und beispielsweise in Organicum, VEB Deutscher Verlag der Wissenschaften, 17. Auflage, Berlin, 1988, Seite 179 ff beschrieben, worauf hiermit in vollen Umfang Bezug genommen wird.

C₁-C₆-Alkyl ist ein linearer oder verzweigter Alkylrest mit 1 bis 6 Kohlenstoffatomen. Beispiele hierfür sind Methyl, Ethyl, n-Propyl, Isopropyl, n-Butyl, 2-Butyl, Isobutyl oder *tert*-Butyl. C₁-C₂-Alkyl ist Methyl oder Ethyl, C₁-C₃-Alkyl ist darüber hinaus n-Propyl oder Isopropyl.

Halogen-C₁-C₄-Alkyl ist beispielsweise Halogenmethyl, Dihalogenmethyl, Trihalogenmethyl, (R)-1-Hatogenethyl, (S)-1-Halogenethyl, 2-Halogenethyl, 1,1-Dihalogenethyl, 2,2-Dihalogenethyl, 2,2,2-Trihalogenethyl, (R)-1-Halogenpropyl, (S)-1-Hatogenpropyl, 2-Halogenpropyl, 3-Halogenpropyl, 1,1-Dihalogenpropyl, 2,2-Dihalogenpropyl, 3,3-Dihalogenpropyl, 3,3,3-Trihalogenpropyl, (R)-2-Halogen-1-methylethyl, (S)-2-Halogen-1-methylethyl, (R)-2,2-Dihalogen-1-methylethyl, (S)-2,2-Dihalogen-1-methylethyl, (R)-1,2-Dihalogen-1-methylethyl, (S)-1,2-Dihalogen-1-methylethyl, (R)-2,2,2-Trihalogen-1-methylethyl, (S)-2,2,2-Trihalogen-l-methylethyl, 2-Halogen-1-(halogenmethyl)ethyl, 1-(Dihalogenmethyl)-2,2-dihalogenethyl, (R)-1-Halogenbutyl, (S)-1-Halogenbutyl, 2-Halogenbutyl, 3-Halogenbutyl, 4-Halogenbutyl, 1,1-Dihalogenbutyl, 2,2-Dihalogenbutyl, 3,3-Dihalogenbutyl, 4,4-Dihalogenbutyl oder 4,4,4-Trihalogenbutyl. Analoges gilt für Halogenalkoxy und Halogenalkylthio.

Hydroxy-C₁-C₄-Alkyl ist beispielsweise Hydroxymethyl, (R)-1-Hydroxyethyl, (S)-1-Hydroxyethyl, 2-Hydroxyethyl, (R)-1-Hydroxypropyl, (S)-1-Hydroxypropyl, 2-Hydroxypropyl, 3-Hydroxypropyl, (R)-2-Hydroxy-1-methylethyl, (S)-2-Hydroxy-1-methylethyl, 2-Hydroxy-1-(hydroxymethyl)ethyl, (R)-1-Hydroxybutyl, (S)-1-Hydroxybutyl, 2-Hydroxybutyl, 3-Hydroxybutyl oder 4-Hydroxybutyl.

Halogen steht im Rahmen der vorliegenden Erfindung für Fluor, Chlor, Brom oder lod. Als Substituent eines Phenylrests sind Fluor und Chlor bevorzugt. Gleiches gilt für Halogenalkyl und Halogenalkoxy.

C₂-C₆-Alkenyl ist ein einfach ungesättigter Kohlenwasserstoffrest mit 2, 3, 4, 5 oder 6 Kohlenstoffatomen, z.B. Vinyl, Allyl (2-Propen-1-yl), 1-Propen-1-yl, 2-Propen-2-yl oder Methallyl (2-Methylprop-2-en-1-yl).

C₆-C₁₂-Aryl ist ein 6- bis 12-gliedriger, insbesondere 6- bis 10-gliedriger, aromatischer zyklischer Rest. Hierzu gehören beispielsweise Phenyl und Naphthyl.

Der Ausdruck "substituiert mit 1 bis 3 oder 1 bis 5 Substituenten, die ausgewählt sind unter ..." meint "substituiert mit 1, 2 oder 3 bzw. 1, 2, 3, 4 oder 5 Substituenten, die ausgewählt sind unter ...", wobei die Substituenten gleich oder verschieden sein können.

Vorzugsweise sind alle genannten Reste R^{x} in den Ausgangs-, Zwischen und Endprodukten gegenüber den Epoxidierungs- und Reduktionsbedingungen stabil. Erforderlichenfalls kann ein labiler Rest aber durch Einführung geeigneter Gruppen zeitweise geschützt werden.

In den erfindungsgemäßen Ausgangs-, Zwischen- und Endrodukten stehen R¹ und R² insbesondere für Phenyl, das 1 bis 3 Substituenten aufweist, die ausgewählt sind unter Halogen, Nitro, C₁-C₄-Alkyl, C₁-C₄-Alkoxy, Phenoxy, Amino, Halogen-C₁-C₂-alkyl und Phenylsulfonyl. Besonders hervorzuheben sind End- und Zwischenprodukte, in denen R¹ und R² unabhängig voneinander für Phenyl stehen, das 1 bis 3 Halogenatome aufweist. Beispielsweise stehen R¹ für 4-Fluorphenyl und R² für 2-Chlorphenyl. Bevorzugt stehen R¹ für 2-Chlorphenyl und R² für 4-Fluorphenyl (wie im Epoxiconazole) oder R¹ für 2-Chlorphenyl und R² für 2,4-Difluorphenyl.

In den erfindungsgemäßen Ausgangs-, Zwischen- und Endprodukten stehen R³ und R⁴ insbesondere für Wasserstoff. Einer weiteren besonderen Ausführungsform zufolge stehen R³ insbesondere für Wasserstoff und R⁴ insbesondere für Mercapto, -S-CN, C₁-C₆-Alkylthio, C₂-C₆-Alkenylthio (z.B. Allylthio) oder C₆-C₁₂-Aryl-C₁-C₃-alkylthio (z.B. Benzylthio), wobei C₂-C₆-Alkenylthio (z.B. Allylthio) 1 bis 3 Substituenten aufweisen kann, die ausgewählt sind unter Halogen, C₁-C₄-Alkyl und Halogen-C₁-C₄-alkyl, und das Aryl (z.B. Phenyl) in C₆-C₁₂-Aryl-C₁-C₃-alkylthio (z.B. Benzylthio) 1 bis 5 Substituenten aufweisen kann, die ausgewählt sind unter Halogen, C₁-C₄-Alkyl und Halogen-C₁-C₄-alkyl.

In den erfindungsgemäßen Ausgangs-, Zwischen- und Endprodukten steht R⁵ insbesondere für Wasserstoff.

Gemäß einer besonderen Ausführungsform betrifft die Erfindung ein Verfahren zur Herstellung von [1,2,4]Triazol-1-ylmethyloxiranen, d.h. X ist ein Stickstoffatom.

Insbesondere ist das Azolylmethyloxiran der Formel (I) eine Verbindung mit der Formel (Ia) 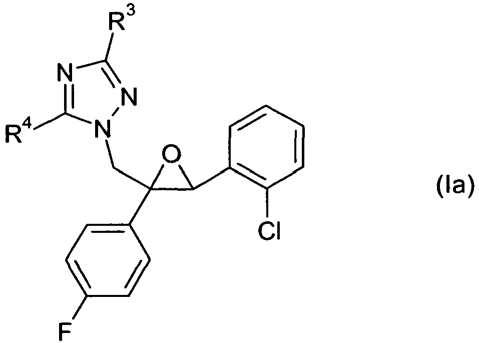 worin R³ und R⁴ wie hier definiert sind und vorzugsweise für Wasserstoff stehen.

Einer weiteren besonderen Ausführungsform zufolge ist das Azolylmethyloxiran der Formel (I) eine Verbindung mit der Formel (Ib) 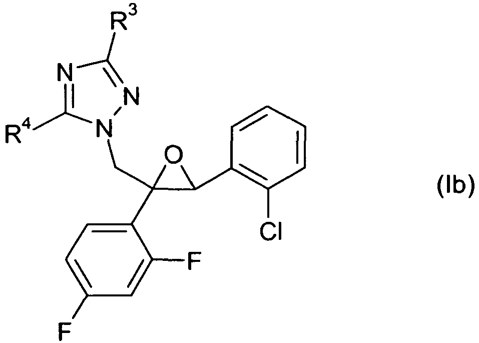 worin R³ und R⁴ wie hier definiert sind und R³ vorzugsweise für Wasserstoff und R⁴ vorzugsweise für Mercapto, -S-CN, C₁-C₆-Alkylthio, C₂-C₆-Alkenylthio (z.B. Allylthio) oder C₆-C₁₂-Aryl-C₁-C₃-alkylthio (z.B. Benzylthio), wobei C₂-C₆-Alkenylthio (z.B. Allylthio) 1 bis 3 Substituenten aufweisen kann, die ausgewählt sind unter Halogen, C₁-C₄-Alkyl und Halogen-C₁-C₄-alkyl, und das Aryl (z.B. Phenyl) in C₆-C₁₂-Aryl-C₁-C₃-alkylthio (z.B. Benzylthio) 1 bis 5 Substituenten aufweisen kann, die ausgewählt sind unter Halogen, C₁-C₄-Alkyl und Halogen-C₁-C₄-alkyl.

Die Ausgangs-, Zwischen- und Endprodukte der vorliegenden Erfindung weisen ein oder mehrere Asymmetriezentren auf. Sie fallen daher als optisch aktive Verbindungen, meist als Enantiomerengemische an. So ist das Racemat aus dem (2R,3S)- und (2S,3R)-Enantiomeren der Verbindung 1-[3-(2-Chlorphenyl)-2-(4-fluorphenyl)-oxiranyl]methyl-1H-[1,2,4]triazol unter dem Common Name "Epoxiconazole" bekannt. Mithin können diese und analoge Verbindung in Form von 4 optisch aktiven Enantiomeren vorliegen, von denen 2 als cis- und 2 als trans-Isomere zu bezeichnen sind. 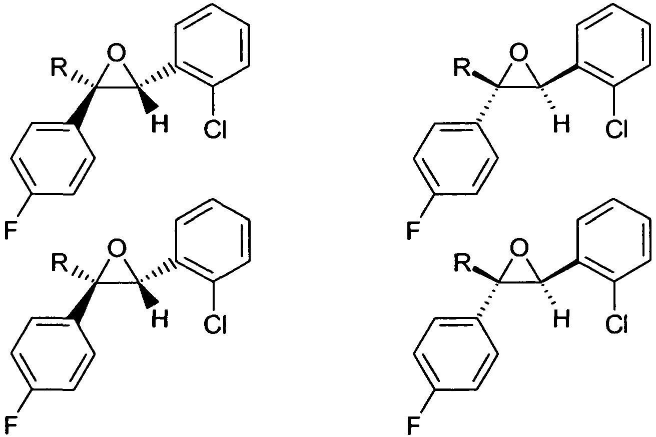 Das erfindungsgemäße Verfahren umfasst die Herstellung sämtlicher dieser Isomeren, sowohl im Gemisch als auch in Reinform. Dazu kann es notwendig sein, die Edukte entsprechend zu wählen, Zwischen- oder Endprodukte aufzutrennen und/oder die Reaktionsbedingungen so zu wählen, dass sie unter Retention der sterischen Konfiguration ablaufen. Auch kann man im Zuge des erfindungsgemäßen Verfahrens gezielt die Bildung einer bestimmten sterischen Konfiguration herbeiführen, beispielsweise durch enantioselektive Epoxidierung.

Die nachfolgend beschriebenen Ausführungsbeispiele sollen die Erfindung näher erläutern, ohne sie zu beschränken.
1. Herstellung von 2,3-Propenalen der Formel (V)
   1.1 Umsetzung von Phenylglyoxal-O,O-acetalen mit Benzylphosphonsäuredialklylestern nach Horner und Emmons
   1.1.1 Herstellung von 2-(4-Fluorphenyl)-3-(2-ch)orphenyl)-propenal
      Zu 42,4 g (0,2 mol) 4-Fluorphenylglyoxal-O,O-dimethylacetal (96 %ig), gelöst in 300 ml trockenem Dimethylformamid, gab man bei 40 °C 27,2 g (0,24 mol) Kalium-tert.-butylat zu. Unter Stickstoffspülung erhitzte man anschließend auf 100-110 °C und tropfte in 60 min 58 g (0,22 mol) (2-Chlorbenzyl)-phosphonsäurediethylester zu. Man rührte noch 15-30 min und prüfte dabei mittels HPLC auf den vollständigen Umsatz von 4-Fluorphenylglyoxal-O,O-dimethylacetal. Anschließend fügte man der Mischung ca. 1000 ml 10 %ige Kochsalzlösung hinzu und extrahierte dreimal mit 200 ml Methylenchlorid. Nach dem Waschen und Trocknen dampfte man das Lösungsmittel ab und versetzte den Rückstand mit 150 ml MeOH und 20 ml 16 %iger Salzsäure. Man vervollständigte die Fällung nach etwa 30 min durch weiteren Wasserzusatz (20-30 ml), saugte ab und wusch mit einer Mischung von Methanol/Wasser (3:1) säurefrei. Nach dem Trocknen erhielt man 48 g 2-(4Fluorphenyl)-3-(2-chlorphenyl)-propenal
      (Ausbeute 91,7 %, Fp. 87-89 °C, 21 % cis-Isomer).
   1.1.2 Herstellung von 2-(Phenyl)-3-(phenyl)-propenal
      Analog zu Beispiel 1.1.1 setzte man Phenylglyoxal-O,O-dimethylacetal mit Benzylphosphonsäurediethylester um (Fp. 90-92 °C).
   1.1.3 Herstellung von 2-(4-Chlorphenyl)-3-(phenylbpropenal
      Analog zu Beispiel 1.1.1 setzte man 4-Chlorphenylglyoxal-O,O-dimethylacetal mit Benzylphosphonsäurediethylester um (Fp. 82-84 °C).
   1.1.4 Herstellung von 2-(4-Fluorphenyl)-3-(2-trifluormethylphenyl)-propenal
      Analog zu Beispiel 1.1.1 setzte man 4-Fluorphenylglyoxal-O,O-dimethylacetal mit (2-Trifluormethylbenzyl)-phosphonsäurediethylester um. Allerdings musste man das Produkt nach der Hydrolyse des Acetals durch Destillation aufarbeiteten werden (Kp. bei 0,25 mbar 112-114 °C).
   1.2 Umsetzung von Phenylglyoxal-O,O-acetalen mit Benzyltriphenylphosphoniumhalogeniden nach Wittig
   1.2.1 Herstellung von 2-(4-Fluorphenyl)-3-(2-chlorphenyl)-propenal
      In eine Lösung von 42,44 g (0,1 mol) 2-Chlorbenzyltriphenylphosphoniumchlorid in 200 ml trockenem Methanol trug man bei 5-10 °C 15,9 g Kalium-tert.-butylat in 75 ml trockenem Methanol ein und gab nach ca. 30 min. 20,2 g (0,095 mol) 4-Fluorphenylglyoxal-O,O-dimethylacetal (96 %ig) in 25 ml Methanol zu. Nach 2 h Rückfluss bei 65 °C ließ man abkühlen, filtrierte vom abgeschiedenen Salz ab und entfernte das Lösungsmittel aus der Mutterlauge. Den Rückstand digerierte man anschließend mehrfach mit Petrolether oder Methyl-tert.-butylether/Cyclohexan (1:3) zur Abtrennung des Triphenylphosphinoxides und dampfte die Lösung wiederum ein. Die Hydrolyse des OO-Acetals und die Ausfällung des Endproduktes erfolgten wie in Beispiel 1.1 beschrieben. Man erhielt 21,1 g 2-(4-Fluorphenyl)-3-(2-chlorphenyl)-propenal.
      (84,5 % Ausbeute, Fp. 79-84 °C, 56 % cis-Isomer).
   1.3 Umsetzung von Phenylglyoxal-O,O-acetalen mit Benzylmagnesiumhalogeniden nach Grignard
   1.3.1 Herstellung von 2-(4-Fluorphenyl)-3-(2-chlorphenyl)-propenal
      Zu 10,6 g (0,44 mol) Magnesiumspänen in 20 ml absolutem Äther fügte man bei 25-35 °C in wenigen Minuten 5 g 2-Chlorbenzylchlorid und 0,2 ml Ethylbromid zu. Nach dem Anspringen der Reaktion tropfte man eine Lösung von 59,8 g (0,369 mol) 2-Chlorbenzylchlorid in 200 ml absolutem Äther zu. Anschließend dekantierte man vom überschüssigen Magnesium ab und legte die Grignardlösung bei 0 °C vor. Danach tropfte man 71 g (0,35 mol) 4-Fluorphenylglyoxal-O,O-dimethylacetal, gelöst in 400 ml trockenem Toluol so zu, dass die Reaktionstemperatur unter 5 °C bleibt, und rührte bis zum vollständigen Umsatz (HPLC-Kontrolle) des Acetals nach (ca. 2h).
      Man goss anschließend auf ca. 50 g Eis und fügte so viel 16 %ige Salzsäure hinzu, dass sich der entstandene Niederschlag gerade löste. Man trennte die ätherische Phase ab und extrahierte noch zweimal mit 100 ml Methyl-tert.-butylether. Nach Abrotieren der Lösungsmittel behandelte man den Rückstand in 300 ml Methanol und 40 ml 16 %iger Salzsäure und vervollständigte die dabei auftretende Ausfällung mit weiterem Wasser (ca. 50 ml). Man erhielt 73 g 2-(4-Fluorphenyl)-3-(2-chlorphenyl)-propenal.
      (69,7 % Ausbeute, Fp. 82-85 °C, 46 % cis-Isomer).
2. Herstellung von Hydroxymethyloxiranen der Formel (III)
   2.1 Getrennte Reaktionsführung von Epoxidierung und Reduktion
      Man löste 125 g (0,479 mol) 2-(4-Fluorphenyl)-3-(2-chlorphenyl)-propenal (DPP) aus Beispiel 1.1.1 in 300 ml Methanol und versetzte mit 2,7 ml (0,05 mol) NaOH (48 %ig). Anschließend tropfte man in 120 min 66 g (0,512 mol) tert.-BuOOH (70 %ig) so zu, dass die Temperatur bei 31 °C blieb, und rührte anschließend noch 90 min zur Vervollständigung der Epoxidation (Gesamtreaktionszeit = 210 min, Umsatz DPP: 98,8 %).
      Anschließend verdünnte man mit 100 ml Toluol und gab 42,3 g Borollösung (12,5 %ige Lösung von NaBH₄ in 40 %iger NaOH, enthaltend 5,29 g NaBH₄) zu. Nach der Aufarbeitung (1 x Extraktion mit 700 ml Toluol und 2 x Waschen mit 150 ml Wasser) erhielt man ein cis/trans-Gemisch des Hydroxymethyloxirans in Toluol mit einer Ausbeute von 92,2 % trans-Isomer.
      Der erhaltene Feststoff enthielt 0,92 % Nebenprodukt A (Difox) und 0,09 %substituiertes 2-(4-Fluorphenyl)-3-(2-chlorphenyl)-propenol.
   2.2 Erfindungsgemäße Reaktionsführung von Epoxidierung und Reduktion
   2.2.1 165 min Gesamtreaktionszeit mit 45 min Nachrühren
      Man verfuhr wie in Beispiel 2.1, reduzierte aber die Gesamtreaktionszeit für die Epoxidierung auf 165 min und rührte lediglich 45 min nach. Bei einem DPP-Umsatz von 94,3 % gab man die Borollösung zu und erhielt nach der Reduktion ein cis/trans-Gemisch des Hydroxymethyloxirans in Toluol mit einer Ausbeute von 92,4 % trans-Isomer.
      Der erhaltene Feststoff enthielt 0,55 % Nebenprodukt A (Difox) und 0,55 % substituiertes 2-(4-Fluorphenyl)-3-(2-chlorphenyl)-propenol.
   2.2.2 150 min Gesamtreaktionszeit mit 30 min Nachrühren
      Man verfuhr wie in Beispiel 2.1, reduzierte aber die Gesamtreaktionszeit für die Epoxidierung auf 150 min und rührte lediglich 30 min nach. Bei einem DPP-Umsatz von 94,3 % gab man die Borollösung zu und erhielt nach der Reduktion ein cis/trans-Gemisch des Hydroxymethyloxirans in Toluol mit einer Ausbeute von 93,1 % trans-Isomer.
      Der erhaltene Feststoff enthielt 0,24 % Nebenprodukt A (Difox) und 1,06 % substituiertes 2-(4-Fluorphenyl)-3-(2-chlorphenyl)-propenol.
   2.2.3 120 min Gesamtreaktionszeit ohne Nachrühren
      Man verfuhr wie in Beispiel 2.1, reduzierte aber die Gesamtreaktionszeit für die Epoxidierung auf 120 min und rührte nicht nach. Bei einem DPP-Umsatz von 92,3 % gab man die Borollösung zu und erhielt nach der Reduktion ein cis/trans-Gemisch des Hydroxymethyloxirans in Toluol mit einer Ausbeute von 91,6 % trans-Isomer.
      Der erhaltene Feststoff enthielt 0,14 % Nebenprodukt A (Difox) und 1,29 % substituiertes 2-(4-Fluorphenyl)-3-(2-chlorphenyl)-propenol.
   2.2.4 Cumolhydroperoxid anstatt tert-Butylhydroperoxid
      Man verfuhr wie in Beispiel 2.2.2, ersetzte aber das tert-Butylhydroperoxid durch Cumolhydroperoxid. Bei einem DPP-Umsatz von 93,5 % gab man die Borollösung zu und erhielt nach der Reduktion ein cis/trans-Gemisch des Hydroxymethyloxirans in Toluol mit einer Ausbeute von 92,1 % trans-Isomer.
      Der erhaltene Feststoff enthielt 0,28 % Nebenprodukt A (Difox) und 0,98 % substituiertes 2-(4-Fluorphenyl)-3-(2-chlorphenyl)-propenol.
   2.2.5 Höhere Katalysatormenge
      Man verfuhr wie in Beispiel 2.1, reduzierte aber die Gesamtreaktionszeit für die Epoxidierung auf 135 min, rührte 30 min nach, erhöhte die Katalysatormenge auf 3,5 ml 50 %ige NaOH und senkte die Reaktionstemperatur nach dem Anspringen bei 31 °C auf 25 °C.
      Bei einem DPP-Umsatz von 92,9 % gab man die Borollösung zu und erhielt nach der Reduktion ein cis/trans-Gemisch des Hydroxymethyloxirans in Toluol mit einer Ausbeute von 90,1 % trans-Isomer.
      Der erhaltene Feststoff enthielt 0,26 % Nebenprodukt A (Difox) und 1,34 % substituiertes 2-(4-Fluorphenyl)-3-(2-chlorphenyl)-propenol.
   2.2.6 Reduktionsmittel in zwei Portionen
      Man verfuhr wie in Beispiel 2.2.2, gab bei einem DPP-Umsatz von 94,9 % aber zur Reduktion die Borolmenge in zwei Portionen zu, nämlich 4,2 g sofort und nach 15 min die restliche Menge (38,1 g). Man erhielt ein cis/trans-Gemisch des Hydroxymethyloxirans in Toluol mit einer Ausbeute von 92,1 % trans-Isomer.
      Der erhaltene Feststoff enthielt 0,21 % Nebenprodukt A (Difox) und 0,64 % substituiertes 2-(4-Fluorphenyl)-3-(2-chlorphenyl)-propenol.
   2.2.7 Tetramethylammoniumhydroxid anstatt NaOH
      Man verfuhr wie in Beispiel 2.2.2, ersetzte aber die NaOH durch 11,4 g (0,05 mol) Tetramethylammoniumhydroxid (40 %ig). Bei einem DPP-Umsatz von 92,2 % bei Zugabe der Borollösung erhielt man cis/trans-Gemisch des Hydroxymethyloxirans in Toluol mit einer Ausbeute von 88,9 % trans-Isomer.
      Der erhaltene Feststoff enthielt 0,16 % Nebenprodukt A (Difox) und 1,14 % substituiertes 2-(4-Fluorphenyl)-3-(2-chlorphenyl)-propenol.
   2.2.8 Vergleichsbeispiel in Analogie zu den Beispielen B und G aus DE 3825586 (EP 352 675):
      Man löste 85 g (0,326 mol) 2-(4-Fluorphenyl)-3-(2-chlorphenyl)-propenal (DPP) aus Beispiel 1.1.1 in 300 ml Methanol und versetzte mit 2,3 ml (0,043 mol) NaOH (50 %ig). Anschließend tropfte man in 30 min 27,7 g (0,794 mol) H₂O₂ (50 %ig) so zu, dass die Temperatur bei 30 ° blieb, und rührte anschließend noch 6 h zur Vervollständigung der Epoxidation (Reaktionszeit = 6,5 h, Umsatz DPP: > 99,5 %).
      Nach der Reduktion mit 35 g Borollösung (12,5 %ige Lösung von NaBH₄ in 40 %iger NaOH) erhielt man nach der Aufarbeitung (1 x Extraktion mit 600 ml Toluol und 2 x Waschen mit 150 ml Wasser) ein cis/trans-Gemisch des Hydroxymethyloxirans), gelöst in Toluol mit einer Ausbeute an trans-Isomer von 63,5 %.
      Der erhaltene Feststoff enthielt 2,69 % Nebenprodukt A (Difox), 0,02 % substituiertes 2-(4-Fluorphenyl)-3-(2-chlorphenyl)-propenol und weitere Nebenprodukte (HPLC).
   2.2.9 Weiteres Vergleichsbeispiel in Analogie zu den Beispielen B und G aus DE 3825586 (EP 352 675):
      Man verfuhr wie in Beispiel 2.2.8, ersetzte aber das H₂O₂ durch 45,3 g (0,352 mol) tert.-Butylhydroperoxid (70 %ig in Wasser). Bei einem DPP-Umsatz von 99,1 % erhielt man cis/trans-Gemisch des Hydroxymethyloxirans in Toluol mit einer Ausbeute von 82,2 % trans-Isomer.
      Der erhaltene Feststoff enthielt 2,45 % Nebenprodukt A (Difox), 0,22 % substituiertes 2-(4-Fluorphenyl)-3-(2-chlorphenyl)-propenol und weitere Nebenprodukte (HPLC).
3. Herstellung von Azolylmethyloxiranen der Formel (I)
   3.1 Triazolylmethyloxiran aus erfindungsgemäßem Hydroxymethyloxiran
      Man versetzte die nach Beispiel 2.2.2 erhaltene Lösung des cis/trans-Hydroxymethyloxirans (130 g (0,471 mol) in 800 ml Toluol) mit 78 g (0,612 mol) N,N-Dimethylcyclohexylamin und trocknete durch azeotrope Destillation. Bei 25 °C gab man anschließend in 1 h 62 g (0,541 mol) Methansulfonsäurechlorid zu. Nach 30 min Nachrühren war die Umsetzung vollständig und man extrahierte die gebildeten Salze mit 2 x 200 ml Wasser. Man dampfte die Toluollösung des Mesyloxymethyloxirans bei Temperaturen unter 80 °C im Vakuum ein und nahm den Rückstand anschließend in 305 ml Dimethylformamid auf. Man erhielt eine Lösung von ca. 160 g (0,45 mol) des Mesyloxymethyloxirans in DMF, die man vorlegte und auf ca. 50 °C erwärmte. Man gab unter starkem Rühren 49 g (0,54 mol) Natrium-1,2,4-triazolid und 0,2 g 1,2,4-Triazol hinzu und erhitzte anschließend auf 70 °C. Nach 4 h Rühren bei 70 °C prüfte man den vollständigen Umsatz des Mesyloxymethyloxirans mittels HPLC. Als der Mesyloxymethyloxiran-Gehalt < 0,2 % war, versetzte man mit 30 ml Methanol und kühlte langsam auf 30 °C. Gleichzeitig tropfte man 350 ml Wasser zu, um den das gebildete Triazolylmethyloxiran auszufällen. Man saugte den erhaltenen Niederschlag ab, wusch 2 x mit einer Mischung von Wasser/MeOH (80/20) und trocknete. Man erhielt ca. 120 g Triazolylmethyloxiran mit einem Fp. von 134,7 °C, das 96,2 % trans-Isomer, 2,7 % sym-Isomer, 0,37 % cis-Isomer und 0,20 % Nebenprodukt A (Difox) enthielt.
   3.2 Imidazolylmethyloxiran aus erfindungsgemäßem Hydroxymethyloxiran
      Man verfuhr wie in Beispiel 3.1, ersetzte aber das Natrium-1,2,4-triazolid und 0,2 g 1,2,4-Triazol durch 46,8 g (0,52 mol) Natrium-imidazolid und 0,2 g Imidazol. Man erhielt ca. 88 g Imidazolylmethyloxiran mit einem Fp. von 122-123 °C, das 96,2 % trans-Isomer, 0,96 % cis-Isomer und 0,23 % Nebenprodukt A (Difox) enthielt.
   3.3 Triazolylmethyloxiran aus unreinem Hydroxymethyloxiran
      Man verfuhr wie in Beispiel 3.1, ersetzte aber das Hydroxymethyloxiran aus Beispiel 2.2.2 mit dem Hydroxymethyloxiran aus Beispiel 2.2.8. Das erhaltene Triazolylmethyloxiran enthielt 1,77 % Nebenprodukt A (Difox) II. Nach einer Umkristallisation aus Cyclohexan konnte man den Gehalt an II auf 0,39 % reduzieren.

## Patentansprüche

1. Verfahren zur Herstellung von Hydroxymethyloxiranen der Formel (III): 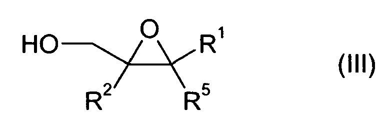 worin
R¹, R² unabhängig voneinander für Phenyl stehen, wobei jeder Phenylrest unabhängig voneinander 1 bis 3 Substituenten aufweisen kann, die ausgewählt sind unter Halogen, Cyano, Nitro, C₁-C₄-Alkyl, Halogen-C₁-C₄-alkyl, Hydroxy, C₁-C₄-Alkoxy, Halogen-C₁-C₄-alkoxy, Mercapto, C₁-C₄-Alkylthio, Halogen-C₁-C₄-alkylthio, Sulfinyl, Sulfonyl, C₁-C₄-Alkylsulfonyl, Phenylsulfonyl, Phenyl, Phenoxy, Amino, C₁-C₄-Alkylamino, Di-C₁-C₃-alkylamino, -NHCO-C₁-C₃-Alkyl, -NHCOO-C₁-C₄-Alkyl, -COO-C₁-C₄-Alkyl und -CONH-C₁-C₄-Alkyl, wobei jeder der Substituenten Phenyl, Phenoxy und Phenylsulfonyl unabhängig voneinander 1 bis 3 Substituenten aufweisen kann, die ausgewählt sind unter Halogen und C₁-C₄-Alkyl; und
R⁵ für Wasserstoff oder Methyl steht,
durch Epoxidierung eines 2,3-Propenals der Formel (V): 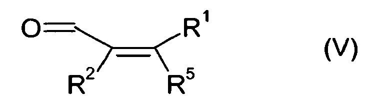 worin R¹, R² und R⁵ wie vorstehend definiert sind,
zu einem Formyloxiran der Formel (IV): 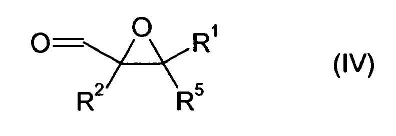 worin R¹, R² und R⁵ wie vorstehend definiert sind,
und Reduktion des Formyloxirans der Formel (IV),
**dadurch gekennzeichnet, dass** mit der Reduktion begonnen wird, solange die Menge an eingesetzter Verbindung der Formel (V) in dem Reaktionsgemisch noch mindestens 2 mol-% beträgt.

2. Verfahren nach Anspruch 1, wobei man zur Epoxidierung die Verbindung der Formel (V) mit einem Hydroperoxid umsetzt.

3. Verfahren nach Anspruch 2, wobei das Hydroperoxid ausgewählt ist unter tert-Butylhydroperoxid, Cumolhydroperoxid, tert-Amylhydroperoxid und Tritylhydroperoxid.

4. Verfahren nach einem der Ansprüche 1 bis 3, wobei die Epoxidierung basenkatalysiert durchgeführt wird.

5. Verfahren nach einem der Ansprüche 1 bis 4, wobei man zur Reduktion ein Alkalimetallborhydrid und eine Base zum Reaktionsgemisch gibt.

6. Verfahren zur Herstellung von Azolylmethyloxiranen der Formel (I): 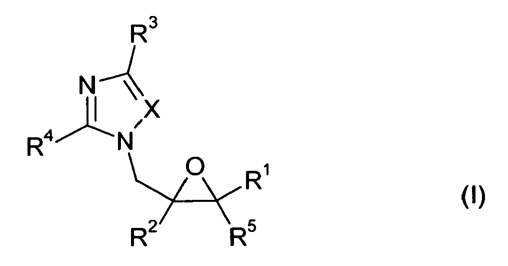 worin
X für N oder CH steht;
R¹, R² unabhängig voneinander für Phenyl stehen, wobei jeder Phenylrest unabhängig voneinander 1 bis 3 Substituenten aufweisen kann, die ausgewählt sind unter Halogen, Cyano, Nitro, C₁-C₄-Alkyl, Halogen-C₁-C₄-alkyl, Hydroxy, C₁-C₄-Alkoxy, Halogen-C₁-C₄-alkoxy, Mercapto, C₁-C₄-Alkylthio, Halogen-C₁-C₄-alkylthio, Sulfinyl, Sulfonyl, C₁-C₄-Alkylsulfonyl, Phenylsulfonyl, Phenyl, Phenoxy, Amino, C₁-C₄-Alkylamino, Di-C₁-C₃-alkylamino, -NHCO-C₁-C₃-Alkyl, -NHCOO-C₁-C₄-Alkyl, -COO-C₁-C₄-Alkyl und -CONH-C₁-C₄-Alkyl, wobei jeder der Substituenten Phenyl, Phenoxy und Phenylsulfonyl unabhängig voneinander 1 bis 3 Substituenten aufweisen kann, die ausgewählt sind unter Halogen und C₁-C₄-Alkyl;
R³, R⁴ unabhängig voneinander für Wasserstoff, Halogen, C₁-C₆-Alkyl, Mercapto, - S-CN, C₁-C₆-Alkylthio, C₂-C₆-Alkenylthio, C₆-C₁₂-Aryl-C₁-C₃-alkylthio oder C₆-C₁₂-Arylthio stehen, wobei C₂-C₆-Alkenylthio 1 bis 3 Substituenten aufweisen kann, die ausgewählt sind unter Halogen, C₁-C₄-Alkyl und Halogen-C₁-C₄-alkyl, und das Aryl in C₆-C₁₂-Aryl-C₁-C₃-alkylthio 1 bis 5 Substituenten aufweisen kann, die ausgewählt sind unter Halogen, C₁-C₄-Alkyl und Halogen-C₁-C₄-alkyl; und
R⁵ für Wasserstoff oder Methyl steht,
durch Epoxidierung eines 2,3-Propenals Formel (V): 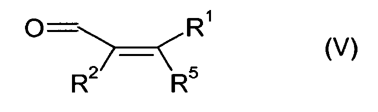 worin R¹, R² und R⁵ wie vorstehend definiert sind,
zu einem Formyloxiran der Formel (IV): 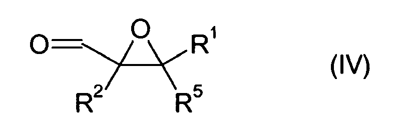 worin R¹, R² und R⁵ wie vorstehend definiert sind,
und Reduktion der Formyloxirans der Formel (IV) zu einem Hydroxymethyloxiran der Formel (III): 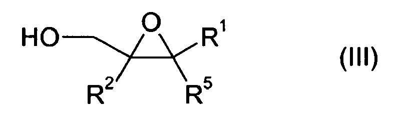 worin R¹, R² und R⁵ wie vorstehend definiert sind,
sowie Einführung der Azolylgruppe in die Verbindung der Formel (III),
**dadurch gekennzeichnet, dass** mit der Reduktion begonnen wird, solange die Menge an eingesetzter Verbindung der Formel (V) in dem Reaktionsgemisch mindestens noch 2 mol-% beträgt.

7. Verfahren nach Anspruch 6, wobei man die Azolylgruppe in die Verbindung der Formel (III) einführt, indem man eine Verbindung der Formel (II) 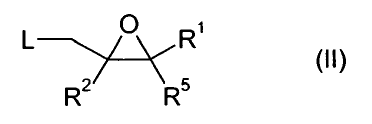 worin R¹, R² und R⁵ wie Anspruch 6 definiert sind und L für eine nukleophil substituierbare Abgangsgruppe steht,
mit einer Verbindung der Formel (VI) 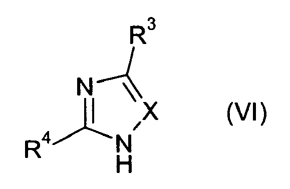 worin R³, R⁴ und X wie Anspruch 6 definiert sind,
oder mit einem Basenadditionssalz der Verbindung der Formel (VI)
umsetzt.

8. Verfahren nach Anspruch 7, wobei L für Halogen, C₁-C₆-Alkyl-SO₂-O- oder Aryl-SO₂-O- steht.

9. Verfahren nach einem der Ansprüche 6 bis 8, wobei X N ist.

10. Verfahren nach einem der Ansprüche 1 bis 9, wobei R¹ und R² unabhängig voneinander für Phenyl stehen, das 1 bis 3 Halogenatome aufweist.

11. Verfahren nach einem der Ansprüche 6 bis 10, wobei das Azolylmethyloxiran der Formel (I) die Verbindung mit der Formel (la) ist, 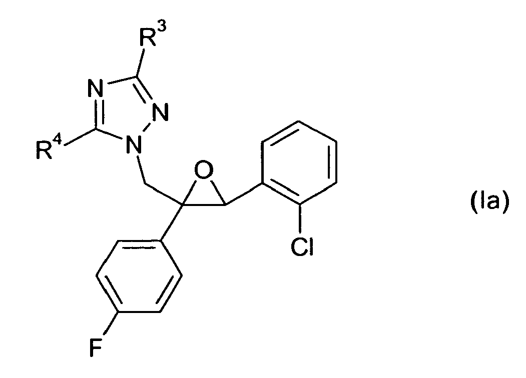 worin R³ und R⁴ wie in Anspruch 6 definiert sind.

12. Verfahren nach einem der Ansprüche 6 bis 11, wobei R³ und R⁴ jeweils für Wasserstoff stehen.

13. Verfahren nach einem der Ansprüche 1 bis 12, wobei R⁵ Wasserstoff ist.

14. Verfahren nach einem der Ansprüche 1 bis 13, wobei mit der Reduktion begonnen wird, wenn die Menge an eingesetzter Verbindung der Formel (V) in dem Reaktionsgemisch weniger als 20 mol-% beträgt.

15. Verfahren nach einem der Ansprüche 1 bis 14, wobei mit der Reduktion begonnen wird, wenn die Menge an eingesetzter Verbindung der Formel (V) in dem Reaktionsgemisch 2,5 bis 15 mol-% beträgt.

## Claims

1. A process for preparing hydroxymethyloxiranes of the formula (III): 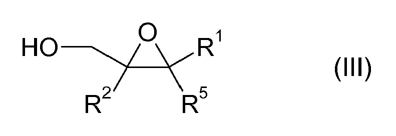 in which
R¹, R² independently of one another are phenyl, where each phenyl radical independently of the other may have 1 to 3 substituents selected from the group consisting of halogen, cyano, nitro, C₁-C₄-alkyl, halo-C₁-C₄-alkyl, hydroxyl, C₁-C₄-alkoxy, halo-C₁-C₄-alkoxy, mercapto, C₁-C₄-alkylthio, halo-C₁-C₄-alkylthio, sulfinyl, sulfonyl, C₁-C₄-alkylsulfonyl, phenylsulfonyl, phenyl, phenoxy, amino, C₁-C₄-alkylamino, di-C₁-C₃-alkylamino, -NHCO-C₁-C₃-alkyl, -NHCOO-C₁-C₄-alkyl, -COO-C₁-C₄-alkyl and -CONH-C₁-C₄-alkyl, where each of the substituents phenyl, phenoxy and phenylsulfonyl independently of the others may have 1 to 3 substituents selected from the group consisting of halogen and C₁-C₄-alkyl; and
R⁵ is hydrogen or methyl,
by epoxidation of a 2,3-propenal of the formula (V): 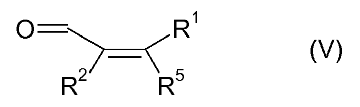 in which R¹, R² and R⁵ are as defined above,
to give a formyloxirane of the formula (IV): 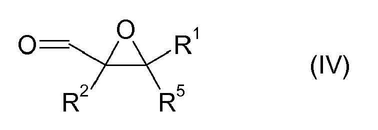 in which R¹, R² and R⁵ are as defined above,
and reduction of the formyloxirane of the formula (IV),
wherein the reduction is started while the amount of the compound of the formula (V) used in the reaction mixture is still at least 2 mol%.

2. The process according to claim 1, where for the epoxidation the compound of the formula (V) is reacted with a hydroperoxide.

3. The process according to claim 2, where the hydroperoxide is selected from the group consisting of tert-butyl hydroperoxide, cumene hydroperoxide, tert-amyl hydroperoxide and trityl hydroperoxide.

4. The process according to any of claims 1 to 3, where the epoxidation is carried out with base catalysis.

5. The process according to any of claims 1 to 4, where for the reduction an alkali metal borohydride and a base are added to the reaction mixture.

6. A process for preparing azolylmethyloxiranes of the formula (I): 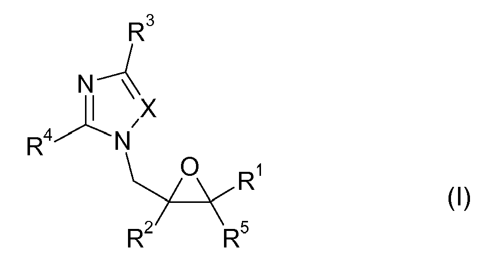 in which
X is N or CH;
R¹, R² independently of one another are phenyl, where each phenyl radical independently of the other may have 1 to 3 substituents selected from the group consisting of halogen, cyano, nitro, C₁-C₄-alkyl, halo-C₁-C₄-alkyl, hydroxyl, C₁-C₄-alkoxy, halo-C₁-C₄-alkoxy, mercapto, C₁-C₄-alkylthio, halo-C₁-C₄-alkylthio, sulfinyl, sulfonyl, C₁-C₄-alkylsulfonyl, phenylsulfonyl, phenyl, phenoxy, amino, C₁-C₄-alkylamino, di-C₁-C₃-alkylamino, -NHCO-C₁-C₃-alkyl, -NHCOO-C₁-C₄-alkyl, -COO-C₁-C₄-alkyl and -CONH-C₁-C₄-alkyl, where each of the substituents phenyl, phenoxy and phenylsulfonyl independently of the others may have 1 to 3 substituents selected from the group consisting of halogen and C₁-C₄-alkyl;
R³, R⁴ independently of one another are hydrogen, halogen, C₁-C₆-alkyl, mercapto, -S-CN, C₂-C₆-alkenylthio, C₁-C₆-alkylthio, C₆-C₁₂-aryl-C₁-C₃-alkylthio or C₆-C₁₂-arylthio, where C₂-C₆-alkenylthio may have 1 to 3 substituents selected from the group consisting of halogen, C₁-C₄-alkyl and halo-C₁-C₄-alkyl, and the aryl in C₆-C₁₂-aryl-C₁-₃-alkylthio may have 1 to 5 substituents selected from the group consisting of halogen, C₁-C₄-alkyl and halo-C₁-C₄-alkyl; and
R⁵ is hydrogen or methyl,
by epoxidation of a 2,3-propenal of the formula (V): 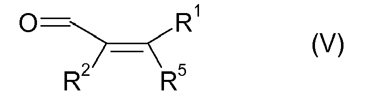 in which R¹, R² and R⁵ are as defined above,
to give a formyloxirane of the formula (IV): 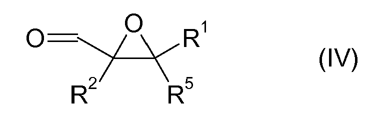 in which R¹, R² and R⁵ are as defined above,
and reduction of the formyloxirane of formula (IV) to give a hydroxymethyloxirane of the formula (III): 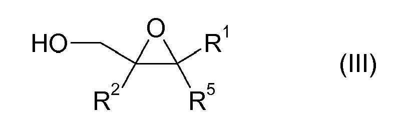 in which R¹, R² and R⁵ are as defined above,
and also the introduction of the azolyl group into the compound of the formula (III),
wherein the reduction is started while the amount of the compound of the formula (V) used in the reaction mixture is still at least 2 mol%.

7. The process according to claim 6, where the azolyl group is introduced into the compound of the formula (III) by reacting a compound of the formula (II) 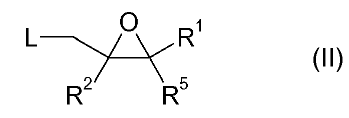 in which R¹, R² and R⁵ are as defined in claim 6 and L is a nucleophilically substitutable leaving group,
with a compound of the formula (VI) 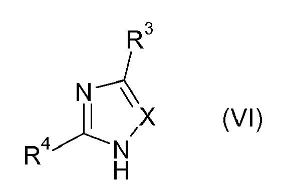 in which R³, R⁴ and X are as defined in claim 6,
or with a base addition salt of the compound of the formula (VI).

8. The process according to claim 7, where L is halogen, C₁-C₆-alkyl-SO₂-O- or aryl-SO₂-O-.

9. The process according to any of claims 6 to 8, where X is N.

10. The process according to any of claims 1 to 9, where R¹ and R² independently of one another are phenyl having 1 to 3 halogen atoms.

11. The process according to any of claims 6 to 10, where the azolylmethyloxirane of
the formula (I) is the compound of the formula (Ia) 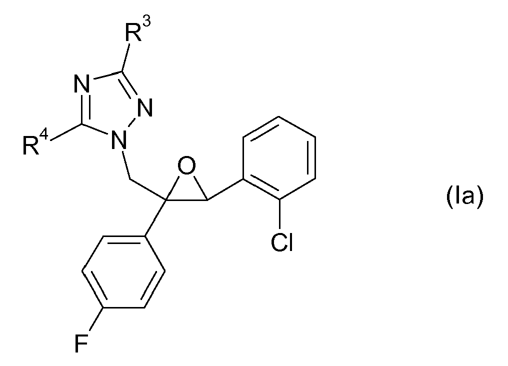 in which R³ and R⁴ are as defined in claim 6.

12. The process according to any of claims 6 to 11, where R³ and R⁴ are each hydrogen.

13. The process according to any of claims 1 to 12, where R⁵ is hydrogen.

14. The process according to any of claims 1 to 13, where the reduction is started when the amount of the compound of the formula (V) used in the reaction mixture is less than 20 mol%.

15. The process according to any of claims 1 to 14, where the reduction is started when the amount of the compound of the formula (V) used in the reaction mixture is from 2.5 to 15 mol%.

## Revendications

1. Procédé pour la préparation d'hydroxyméthyloxiranes de formule (III) _{:} 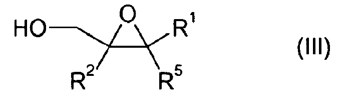 où
R¹, R² indépendamment l'un de l'autre, représentent phényle, chaque radical phényle pouvant présenter, indépendamment l'un de l'autre, 1 à 3 substituants, qui sont choisis parmi halogène, cyano, nitro, C₁-C₄-alkyle, halogéno-C₁-C₄-alkyle, hydroxy, C₁-C₄-alcoxy, halogéno-C₁-C₄-alcoxy, mercapto, C₁-C₄-alkylthio, halogéno-C₁-C₄-alkylthio, sulfinyle, sulfonyle, C₁-C₄-alkylsulfonyle, phénylsulfonyle, phényle, phénoxy, amino, C₁-C₄-alkylamino, di-C₁-C₃-alkylamino, -NHCO-C₁-C₃-alkyle, -NHCOO-C₁-C₄-alkyle, -COO-C₁-C₄-alkyle et -CONH-C₁-C₄-alkyle, chacun des substituants phényle, phénoxy et phénylsulfonyle pouvant présenter, indépendamment l'un de l'autre, 1 à 3 substituants, qui sont choisis parmi halogène et C₁-C₄-alkyle ; et
R⁵ représente hydrogène ou méthyle,
par époxydation d'un 2,3-propénal de formule (V) : 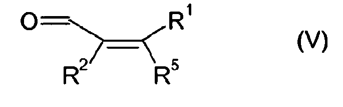 dans laquelle R¹, R² et R⁵ sont tels que définis ci-dessus, en un formyloxirane de formule (IV) _{:} 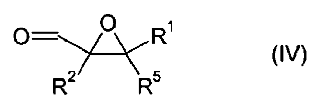 dans laquelle R¹, R² et R⁵ sont tels que définis ci-dessus,
et réduction du formyloxirane de formule (IV), **caractérisé en ce qu'**on démarre la réduction tant que la quantité de composé de formule (V) utilisé dans le mélange réactionnel représente encore au moins 2% en mole.

2. Procédé selon la revendication 1, en transformant pour l'époxydation le composé de formule (V) avec un hydroperoxyde.

3. Procédé selon la revendication 2, l'hydroperoxyde étant choisi parmi l'hydroperoxyde de tert-butyle, l'hydroperoxyde de cumol, l'hydroperoxyde de tert-amyle et l'hydroperoxyde de trityle.

4. Procédé selon l'une quelconque des revendications 1 à 3, l'époxydation étant réalisée sous catalyse par une base.

5. Procédé selon l'une quelconque des revendications 1 à 4, en ajoutant, pour la réduction, un borohydrure de métal alcalin et une base au mélange réactionnel.

6. Procédé pour la préparation d'azolylméthyloxiranes de formule (I) : 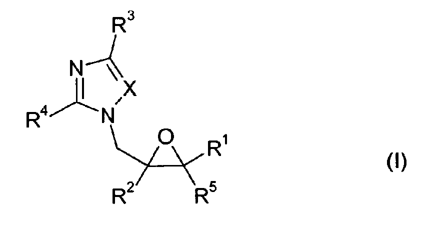 où
X représente N ou CH ;
R¹, R² indépendamment l'un de l'autre, représentent phényle, chaque radical phényle pouvant présenter, indépendamment l'un de l'autre, 1 à 3 substituants, qui sont choisis parmi halogène, cyano, nitro, C₁-C₄-alkyle, halogéno-C₁-C₄-alkyle, hydroxy, C₁-C₄-alcoxy, halogéno-C₁-C₄-alcoxy, mercapto, C₁-C₄-alkylthio, halogéno-C₁-C₄-alkylthio, sulfinyle, sulfonyle, C₁-C₄-alkylsulfonyle, phénylsulfonyle, phényle, phénoxy, amino, C₁-C₄-alkylamino, di-C₁-C₃-alkylamino, -NHCO-C₁-C₃-alkyle, -NHCOO-C₁-C₄-alkyle, -COO-C₁-C₄-alkyle et -CONH-C₁-C₄-alkyle, chacun des substituants phényle, phénoxy et phénylsulfonyle pouvant présenter, indépendamment l'un de l'autre, 1 à 3 substituants, qui sont choisis parmi halogène et C₁-C₄-alkyle ;
R³, R⁴ représentent, indépendamment l'un de l'autre, hydrogène, halogène, C₁-C₆-alkyle, mercapto, -S-CN, C₁-C₆-alkylthio, C₂-C₆-alcénylthio, C₆-C₁₂-aryl-C₁-C₃-alkylthio ou C₆-C₁₂-arylthio, où C₂-C₆-alcénylthio peut présenter 1 à 3 substituants, qui sont choisis parmi halogène, C₁-C₄-alkyle et halogéno-C₁-C₄-alkyle, et aryle dans C₆-C₁₂-aryl-C₁-C₃-alkylthio peut présenter 1 à 5 substituants, qui sont choisis parmi halogène, C₁-C₄-alkyle et halogéno-C₁-C₄-alkyle ; et
R⁵ représente hydrogène ou méthyle,
par époxydation d'un 2,3-propénal de formule (V) : 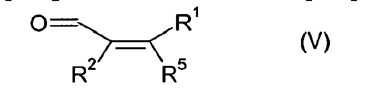 dans laquelle R¹, R² et R⁵ sont tels que définis ci-dessus, en un formyloxirane de formule (IV) _{:} 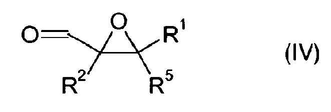 dans laquelle R¹, R² et R⁵ sont tels que définis ci-dessus,
et réduction du formyloxirane de formule (IV) en un hydroxyméthyloxirane de formule (III) _{:} 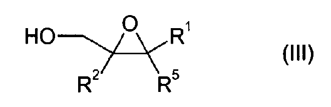 dans laquelle R¹, R² et R⁵ sont tels que définis ci-dessus,
ainsi qu'introduction du groupe azolyle dans le composé de formule (III), **caractérisé en ce qu'**on démarre la réduction tant que la quantité de composé de formule (V) utilisé dans le mélange réactionnel représente encore au moins 2% en mole.

7. Procédé selon la revendication 6, dans lequel on introduit le groupe azolyle dans le composé de formule (III) en ce qu'on transforme un composé de formule (II) 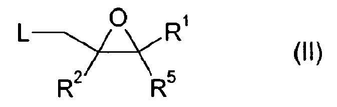 dans laquelle R¹, R² et R⁵ sont définis comme dans la revendication 6 et L représente un groupe partant pouvant être substitué de manière nucléophile,
avec un composé de formule (VI) 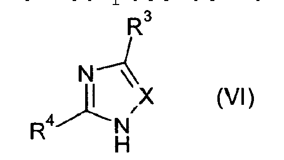 dans laquelle R³, R⁴ et X sont définis comme dans la revendication 6,
ou avec un sel d'addition de base du composé de formule (VI).

8. Procédé selon la revendication 7, L représentant halogène, C₁-C₆-alkyl-SO₂-O- ou aryl-SO₂-O-.

9. Procédé selon l'une quelconque des revendications 6 à 8, X représentant N.

10. Procédé selon l'une quelconque des revendications 1 à 9, R¹ et R² représentant, indépendamment l'un de l'autre, phényle, qui présente 1 à 3 atomes d'halogène.

11. Procédé selon l'une quelconque des revendications 6 à 10, l'azolylméthyloxirane de formule (I) étant le composé de formule (Ia), 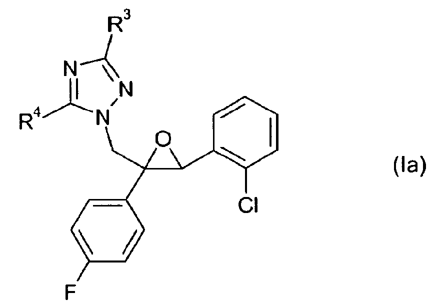 dans laquelle R³ et R⁴ sont définis comme dans la revendication 6.

12. Procédé selon l'une quelconque des revendications 6 à 11, R³ et R⁴ représentant chacun hydrogène.

13. Procédé selon l'une quelconque des revendications 1 à 12, R⁵ représentant hydrogène.

14. Procédé selon l'une quelconque des revendications 1 à 13, la réduction étant démarrée lorsque la quantité de composé de formule (V) utilisé dans le mélange réactionnel représente moins de 20% en mole.

15. Procédé selon l'une quelconque des revendications 1 à 14, la réduction étant démarrée lorsque que la quantité de composé de formule (V) utilisé dans le mélange réactionnel représente 2,5 à 15% en mole.
